# EUROPÄISCHE PATENTSCHRIFT

(11) **EP 4 033 978 B1**
(45) Veröffentlichungstag und Bekanntmachung des Hinweises auf die Patenterteilung: **04.03.2026**
(21) Anmeldenummer: 20804468.5
(22) Anmeldetag: 04.11.2020
(51) Int. Cl.: A61B 6/00, A61K 49/04, A61B 6/03, A61B 6/50

(54) **KONTRASTMITTELBASIERTE GEFÄSSDARSTELLUNG**
CONTRAST AGENT-BASED VASCULAR IMAGING
IMAGERIE VASCULAIRE BASÉE SUR UN AGENT DE CONTRASTE

(30) Priorität: 29.11.2019 DE 102019218587
(43) Veröffentlichungstag der Anmeldung: 03.08.2022
(73) Patentinhaber: Siemens Healthineers AG, 91301 Forchheim (DE); Bayer AG, 51373 Leverkusen (DE)
(72) Erfinder: FLOHR, Thomas, 91486 Uehlfeld (DE); JOST, Gregor, 10318 Berlin (DE); PIETSCH, Hubertus, 14532 Kleinmachnow (DE); SCHMIDT, Bernhard, 90766 Fürth (DE)
(74) Vertreter: Siemens Healthineers Patent Attorneys
(86) Internationale Anmeldenummer: PCT/EP2020/080915
(87) Internationale Veröffentlichungsnummer: WO 2021/104814

(56) Entgegenhaltungen:
- WO-A1-2016/172256
- WO-A1-2017/027547
- US-A1- 2008 137 803

## Beschreibung

Die Erfindung betrifft ein Röntgenkontrastmittel. Zudem betrifft die Erfindung ein Röntgenbildgebungsverfahren, bei dem das genannte Röntgenkontrastmittel Anwendung findet. Weiterhin betrifft die Erfindung eine Bildrekonstruktionseinrichtung. Ferner betrifft die Erfindung ein Röntgenbildgebungssystem.

Mit Hilfe moderner bildgebender Verfahren werden häufig zwei- oder dreidimensionale Bilddaten erzeugt, die zur Visualisierung eines abgebildeten Untersuchungsobjekts und darüber hinaus auch für weitere Anwendungen genutzt werden können.

Häufig basieren die bildgebenden Verfahren auf der Erfassung von Röntgenstrahlung, wobei sogenannte Projektionsmessdaten erzeugt werden. Beispielsweise können Projektionsmessdaten mit Hilfe eines Computertomographie-Systems (CT-Systems) akquiriert werden.

Bei einer Röntgenbildaufnahme werden häufig Kontrastmittel eingesetzt, die dem Patienten injiziert werden, um den Kontrast der Bildaufnahme zu erhöhen und damit eine Diagnose zu erleichtern. Ein Beispiel für die Anwendung von Kontrastmitteln ist die Darstellung von Gefäßen mit Röntgenverfahren. Röntgenverfahren können dabei mit konventionellen Systemen, C-Bogensystemen, Angiographie-Systemen oder CT-Systemen durchgeführt werden. Herkömmlich wird bei einer solchen Bildgebung Iod als Röntgenkontrastmittel eingesetzt.

Allerdings tritt bei der Angiographie mit Iod das Problem auf, dass Knochen und Verkalkungen sowie teilkalzifizierte Plaques in den Gefäßen ähnliche Dichtewerte aufweisen wie das kontrastmittelgefüllte Gefäß und so insbesondere bei komplexen anatomischen Strukturen, wie zum Beispiel im Schädelbereich, Iodgefüllte Gefäße, Knochen und Verkalkungen sich in den Gefäßen überlagern und daher in der Bilddarstellung nicht mehr exakt voneinander getrennt werden können. Daher lässt sich insbesondere bei Gefäßen mit geringem Durchmesser deren Gefäßweite, auch als Lumen bezeichnet, bei starker Verkalkung mit Hilfe der CT-Angiographie oft nicht mehr ermitteln. Dieses Phänomen stellt eine Einschränkung für die Anwendung der CT-Angiographie bei Koronararterien und peripheren Beingefä-ßen dar.

Herkömmlich wurde versucht, durch Subtraktion einer Voraufnahme eines aufzunehmenden Bereichs, die ohne Kontrastmittel durchgeführt wurde, Knochen und Verkalkungen bzw. teilkalzifizierte Plaques in Gefäßen von den durch Iod-Kontrastmittel sichtbar gemachten Bereichen zu trennen. Allerdings ergeben sich aufgrund des zeitlichen Versatzes zwischen Voraufnahme und Hauptaufnahme Probleme der Registrierung aufgrund von Patientenbewegungen. Weiterhin wird durch die zusätzliche Bildaufnahme die Strahlendosis für den Patienten erhöht.

Auch die Anwendung von softwarebasierten Verfahren zur Trennung von Bildinformation, welche die Gefäße betrifft und Bildinformation, welche Knochen zuzuordnen ist, ist fehlerbehaftet. Diese Fehler treten insbesondere bei komplexen Darstellungen, wie zum Beispiel der Schädelbasis, auf. Überdies ist eine Eliminierung (teil)kalzifizierter Plaques aus der Darstellung von Gefäßen aufgrund des Blooming-Effekts nicht möglich. Denn die teilkalzifizierten Plaques werden aufgrund des Blooming-Effekts größer dargestellt als sie in Wirklichkeit sind. Werden die Darstellungen der Plaques aus dem Bild entfernt, so bleiben aufgrund der von den Plaques vorher abgedeckten zu großen Flächen Bereiche zurück, über die keine Bildinformationen vorliegen.

Eine Möglichkeit, Knochen und iodbehaftete Kontrastmittelpixel zu trennen, besteht in der Anwendung von Dual-Energie oder Multi-Energie-Bildgebungsverfahren. Dabei werden Bildaufnahmen ein- und desselben Untersuchungsbereiches mit mindestens zwei unterschiedlichen mittleren Röntgenenergien durchgeführt. Allerdings treten bei einfachen Varianten, bei denen Pixel dem Kontrastmittel oder Knochenmaterial durch Klassifikation zugeordnet werden, ähnliche Probleme auf, wie sie bei der Anwendung softwarebasierter Verfahren auftreten. Es kommt ebenfalls zu fehlerhafter Trennung von Iodbehafteten Bereichen und von mit Plaques behafteten Bereichen, insbesondere bei komplexen Gefäßstrukturen. Ebenso kommt es auch bei dieser Vorgehensweise zur vergrößerten Darstellung der kalzifizierten Bereiche. Werden diese Bereiche anschließend extrahiert, um die Gefäße sichtbar zu machen, ist unklar, wie die durch die vergrößerte Darstellung abgedeckten Bereiche im Bild dargestellt werden sollen.

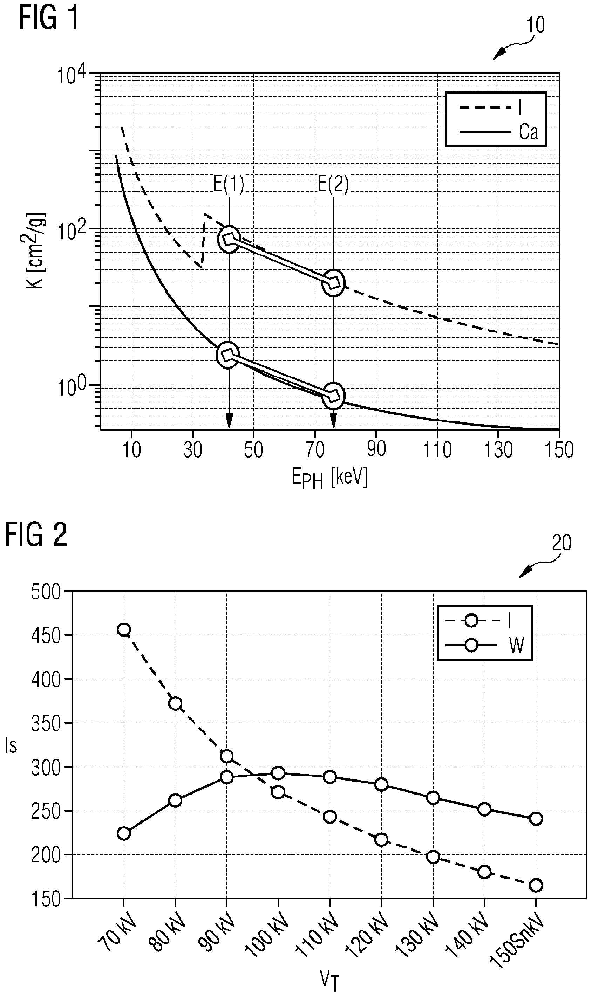

Bei einem anderen relativ neuen Verfahren wird eine Dual-Energie-Bildaufnahme dazu genutzt, auf Basis dieser Aufnahme zwei unterschiedliche Bilder zu berechnen, wobei eines der beiden Bilder die mit Calcium behafteten Strukturen wiedergibt und das andere der beiden Bilder Bereiche darstellt, die mit Kontrastmittel beaufschlagt sind. In dem Kontrastmittelbild sind mithin weder Knochen noch Verkalkungen der Gefäße erkennbar. Zwar werden auf diese Weise vergrößerte Darstellungen der Kalzifizierungen im Bild vermieden und es liegen Informationen über die sonst von den vergrößerten Strukturen verdeckten Bereiche vor, allerdings besteht weiterhin das Problem, dass bisher verwendete Kontrastmittel, wie zum Beispiel Iod und Knochen oder Kalzifizierungen bezüglich ihres spektralen Absorptionsverhaltens im Energiebereich von Röntgenaufnahmen (40 keV bis 140 keV) sehr ähnlich sind. D.h., bei beiden Materialien nimmt die Röntgenabsorption zu niedrigeren Energien hin stark zu. In FIG 1 ist die Absorption für eine Aufnahme mit niedriger Energie (bezeichnet mit E(1)) und eine Aufnahme mit hoher Energie (bezeichnet mit E(2)) dargestellt. Aufgrund des ähnlichen Verhaltens des herkömmlichen Kontrastmittels Iod und von Calcium zeigen die resultierenden Materialbilder ein sehr hohes Bildrauschen und eine unpräzise Materialtrennung. Zwar kann das Problem des hohen Bildrauschens und der unpräzisen Materialtrennung durch eine signifikante Erhöhung der Strahlendosis behoben werden, allerdings bringt diese Vorgehensweise eine erhöhte gesundheitliche Belastung des Patienten mit sich und lässt sich bei machen Untersuchungsarten, wie zum Beispiel der Koronarangiographie aufgrund technischer Limitierungen nicht realisieren.

In WO 2016/ 172 256 A1 wird die Anwendung eines Darmkontrastmittels beschrieben. Das Darmkontrastmittel weist eine Suspension mit kapselartigen Partikeln mit einem mit Gas oder einem Vakuum beaufschlagten Innenraum auf. Das spektrale Absorptionsverhalten des Darmkontrastmittels wird dabei so gewählt, dass es sich von dem spektralen Absorptionsverhalten eines zweiten Kontrastmittels, welches in einem Nachbarbereich, beispielsweise in Blutgefäßen oder in einem Nachbarorgan, eingesetzt wird, stark unterscheidet.

In US 2008/ 0 137 803 A1 wird ein medizinisches Bildgebungssystem beschrieben, welches dazu eingerichtet ist, Körperbereiche, welche mit Kontrastmitteln mit unterschiedlichen Absorptionskanten für Röntgenstrahlung beaufschlagt sind, voneinander getrennt darzustellen.

In WO 2017/ 027 547 A1 wird ein System für eine bildgesteuerte Bestrahlungstherapie beschrieben. Zur Reduktion der Strahlendosis werden die für die Bilddarstellung und die Bestrahlungstherapie genutzten Kontrastmittel so gewählt, dass sie eine starke Absorption für die zur Therapie genutzte Strahlung aufweisen und eine Bilddarstellung und Abgrenzung eines zu therapierenden Körperbereichs von seiner Umgebung ermöglichen.

Es besteht also das Problem, qualitativ gute Gefäßdarstellungen mit geringer Strahlendosis zu realisieren.

Diese Aufgabe wird durch ein Röntgenkontrastmittel zur Darstellung von mit dem Röntgenkontrastmittel durchfluteten Blutgefäßen gemäß Patentanspruch 1, ein Röntgenbildgebungsverfahren zur Darstellung von mit einem Röntgenkontrastmittel durchfluteten Blutgefäßen gemäß Patentanspruch 5, eine Bildrekonstruktionseinrichtung gemäß Patentanspruch 9 und ein Röntgenbildgebungssystem gemäß Patentanspruch 10 gelöst.

Das erfindungsgemäße Röntgenkontrastmittel zur Darstellung von mit dem Röntgenkontrastmittel durchfluteten Blutgefäßen weist eine Röntgenabsorption auf, deren Änderung zwischen mindestens zwei unterschiedlichen Röntgenphotonenenergien sich signifikant von der Änderung der Röntgenabsorption von Calcium zwischen den mindestens zwei unterschiedlichen Röntgenphotonenenergien unterscheidet.

Idealerweise sollte die Absorption des erfindungsgemäßen Röntgenkontrastmittels zwischen den mindestens zwei unterschiedlichen Röntgenphotonenenergien nahezu konstant bleiben. Unter "signifikant" soll in diesem Zusammenhang verstanden werden, dass die Änderung weniger als die Hälfte der Änderung von Calcium bei den gewählten unterschiedlichen Röntgenphotonenenergien beträgt.

Ist die Röntgenabsorption des Röntgenkontrastmittels für die mindestens zwei Röntgenphotonenenergien nicht signifikant unterschiedlich, so wird vorteilhaft erreicht, dass sich das erfindungsgemäße Röntgenkontrastmittel damit hinsichtlich seines Absorptionsverhaltens in Abhängigkeit von der Photonenenergie gegenüber insbesondere in der Angiographie auftretenden Materialien wie Calcium deutlich unterscheidet.

Vorteilhaft lässt sich das spektral abweichende Verhalten des erfindungsgemäßen Kontrastmittels dazu nutzen, von dem Kontrastmittel durchflutete Bereiche von anderen Bildbereichen, die kalzifiziert oder teilkalzifiziert sind, getrennt dazustellen. Insbesondere bei der angiographischen Darstellung von Gefäßen ergeben sich genauere Werte für die Öffnungsweite der dargestellten Gefäße und die Genauigkeit der Darstellung ist im Vergleich zu herkömmlich angewendeten Kontrastmitteln verbessert. Mithin ist das erfindungsgemäße Röntgenkontrastmittel bei der bildlichen Darstellung von Blutgefäßen vorteilhaft anwendbar, da damit der Innendurchmesser von Gefäßen besonders exakt dargestellt werden kann.

Bei dem erfindungsgemäßen Röntgenbildgebungsverfahren zur Darstellung von mit einem Röntgenkontrastmittel durchfluteten Blutgefäßen erfolgt zunächst eine Wahl eines erfindungsgemä-ßen Kontrastmittels. Weiterhin werden Röntgenrohdaten von einem von dem Kontrastmittel durchfluteten Bereich eines Untersuchungsobjekts mit Hilfe eines Multi-Energie-Aufnahmeverfahrens erfasst. Das erfindungsgemäße Röntgenbildgebungsverfahren kann als Computer-implementiertes Verfahren auf Basis der erfassten Daten durchgeführt werden.

Auf Basis der Röntgenrohdaten erfolgt eine Materialzerlegung in Daten, die entweder dem erfindungsgemäßen Kontrastmittel oder Calcium zuzuordnen sind.

Bei der prinzipiell bekannten Materialzerlegung wird von der Überlegung ausgegangen, dass ein mittels einer Röntgenbildaufnahmevorrichtung gemessener Röntgenschwächungswert als Linearkombination von Röntgenschwächungswerten von sogenannten Basismaterialien bezüglich der besagten Röntgenquantenenergieverteilung bzw. Röntgenphotonenenergie beschrieben werden kann. Gemessene Röntgenschwächungswerte ergeben sich aus den wenigstens zwei Rohdatensätzen bzw. daraus rekonstruierten Bilddatensätzen zu unterschiedlichen Röntgenquantenenergieverteilungen. Material bzw. Basismaterial sind bei der erfindungsgemäßen Anwendung zum einen Calcium und zum anderen das erfindungsgemäße Röntgenkontrastmittel. Die Röntgenschwächung eines Basismaterials in Abhängigkeit von der Energie der Röntgenstrahlung ist grundsätzlich bekannt oder kann durch vorherige Messungen an Phantomen bestimmt werden und in Form von Tabellen zum Abruf im Rahmen der Materialzerlegung hinterlegt sein. Ergebnis der Materialzerlegung ist eine räumliche Dichteverteilung der wenigstens zwei Materialien, d.h. des erfindungsgemäßen Kontrastmittels und Calcium im Patienten, woraus sich für jedes Volumenelement in der abzubildenden Körperregion des Patienten die Basismaterialanteile bzw. die Basismaterialienkombination ermitteln lassen. Die Materialzerlegung kann sowohl direkt die Rohdaten betreffen als auch anhand von rekonstruierten Bilddaten erfolgen. Jedenfalls werden im Rahmen des Verfahrens mindestens zwei Bilddatensätze auf Basis spektralzerlegter Daten, seien es Rohdaten oder Bilddaten erzeugt: Die mindestens zwei Bilddatensätze umfassen einen ersten Bilddatensatz, welcher einen durch das erfindungsgemäße Kontrastmittel beaufschlagten ersten Bildbereich darstellt, und einen zweiten Bilddatensatz, welcher einen vorzugsweise zu dem ersten Bildbereich komplementären zweiten Bildbereich darstellt, in dem calciumhaltige Strukturen sichtbar gemacht werden.

Für den Fall einer komplementären Darstellung des ersten und des zweiten Bilddatensatzes können durch das erfindungsgemäße Kontrastmittel beaufschlagte Bereiche und calciumhaltige Strukturen gemeinsam in einem Bild, beispielsweise durch eine Überlagerung der beiden Bilddatensätze, veranschaulicht werden, wobei die Relativposition der unterschiedlichen Strukturen bzw. Materialien sowie die räumliche Trennung bzw. Grenzflächen zwischen diesen unterschiedlichen Strukturen bzw. Materialien gut zu erkennen sind.

Liegt eine Durchmischung der durch die beiden Bilddatensätze dargestellten unterschiedlichen Materialien vor, so kann auch zur getrennten Veranschaulichung der unterschiedlichen Materialien eine jeweils gesonderte Darstellung des ersten und des zweiten Bilddatensatzes in zwei separaten Bildern erfolgen.

Das erfindungsgemäße Röntgenbildgebungsverfahren ermöglicht eine getrennte Darstellung von calciumhaltigen und durch das erfindungsgemäße Kontrastmittel beaufschlagten Bildbereichen. Damit wird beispielsweise eine präzisere Darstellung des Innendurchmessers von Gefäßen erreicht, was zu einer zuverlässigeren Diagnose auf Basis von angiographischen Bilddaten beiträgt. Außerdem kann die Röntgendosis aufgrund der unterschiedlichen spektralen Absorptionseigenschaften des erfindungsgemäßen Kontrastmittels und der übrigen darzustellenden Materialien niedriger als bei herkömmlichen Bildgebungsverfahren gewählt werden.

Die erfindungsgemäße Bildrekonstruktionseinrichtung weist eine Ermittlungseinheit zum Ermitteln von mindestens zwei unterschiedlichen Röntgenphotonenenergien, bei denen sich ein erfindungsgemäßes Kontrastmittel signifikant von der Änderung der Röntgenabsorption von Calcium zwischen den mindestens zwei unterschiedlichen Röntgenphotonenenergien unterscheidet, auf.

Die Wahl der Energiewerte kann im Rahmen eines Multi-Energie-Aufnahmeverfahrens bei der Wahl der Energien bzw. mittleren Energiewerte der zur Bildgebung verwendeten Röntgenquellen berücksichtigt werden. Werden zählende Detektoren zur Erfassung der Röntgenstrahlung verwendet, so können Energieschwellen bzw. Intervalle so gewählt werden, dass die genannten Energiewerte umfasst sind.

Teil der erfindungsgemäßen Bildrekonstruktionseinrichtung ist auch eine Rohdatenempfangseinheit zum Empfangen von Röntgenrohdaten von einem von dem Kontrastmittel zumindest teilweise durchfluteten Bereich eines Untersuchungsobjekts mit Hilfe eines Multi-Energie-Aufnahmeverfahrens.

Die erfindungsgemäße Bildrekonstruktionseinrichtung umfasst auch eine Zerlegungseinheit zum Durchführen einer Materialzerlegung auf Basis der Röntgenrohdaten bezüglich des Kontrastmittels und Calcium eine Rekonstruktionseinheit zum Rekonstruieren von mindestens zwei Bilddatensätzen auf Basis der Materialzerlegung. Grundsätzlich sind solche Materialzerlegungen bei der Bilddarstellung mehrerer Materialien mit Hilfe der Dual-Energie-Bildgebung oder Multi-Energie-Bildgebung, wie bereits erläutert, bekannt.

Die mindestens zwei Bilddatensätze umfassen einen ersten Bilddatensatz, welcher einen durch das Kontrastmittel beaufschlagten ersten Bildbereich darstellt, und einen zweiten Bilddatensatz, welcher vorzugsweise einen zu dem ersten Bildbereich komplementären zweiten Bildbereich darstellt. Die erfindungsgemäße Bildrekonstruktionseinrichtung teilt die Vorteile des erfindungsgemäßen Röntgenbildgebungsverfahrens.

Das erfindungsgemäße Röntgenbildgebungssystem weist eine erfindungsgemäße Bildrekonstruktionseinheit auf. Das erfindungsgemäße Röntgenbildgebungssystem kann vorzugsweise eine CT-Angiographieeinrichtung umfassen. Besonders bei der bildlichen Darstellung von Blutgefäßen ist das erfindungsgemäße Röntgenkontrastmittel vorteilhaft anwendbar, da damit der Innendurchmesser von Gefäßen besonders exakt dargestellt werden kann.

Die wesentlichen Komponenten der erfindungsgemäßen Bildrekonstruktionseinrichtung können zum überwiegenden Teil in Form von Softwarekomponenten ausgebildet sein. Dies betrifft insbesondere die Zerlegungseinheit und die Rekonstruktionseinheit der erfindungsgemäßen Bildrekonstruktionseinrichtung. Grundsätzlich können diese Komponenten aber auch zum Teil, insbesondere wenn es um besonders schnelle Berechnungen geht, in Form von softwareunterstützter Hardware, beispielsweise FPGAs oder dergleichen, realisiert sein. Ebenso können die benötigten Schnittstellen, beispielsweise wenn es nur um eine Übernahme von Daten aus anderen Softwarekomponenten geht, als Softwareschnittstellen ausgebildet sein. Sie können aber auch als hardwaremäßig aufgebaute Schnittstellen ausgebildet sein, die durch geeignete Software angesteuert werden.

Eine weitgehend softwaremäßige Realisierung hat den Vorteil, dass auch schon bisher verwendete medizintechnische Bildgebungssysteme bzw. Bildrekonstruktionseinrichtungen auf einfache Weise durch ein Software-Update nachgerüstet werden können, um auf die erfindungsgemäße Weise zu arbeiten. Insofern wird die Aufgabe auch durch ein entsprechendes Computerprogrammprodukt mit einem Computerprogramm gelöst, welches direkt in eine Speichereinrichtung eines Röntgenbildgebungssystems ladbar ist, mit Programmabschnitten, um die durch Software realisierbaren Schritte des erfindungsgemäßen Röntgenbildgebungsverfahrens auszuführen, wenn das Programm in dem Röntgenbildgebungssystem ausgeführt wird. Ein solches Computerprogrammprodukt kann neben dem Computerprogramm gegebenenfalls zusätzliche Bestandteile wie z. B. eine Dokumentation und/oder zusätzliche Komponenten, auch Hardware-Komponenten, wie z.B. Hardware-Schlüssel (Dongles etc.) zur Nutzung der Software, umfassen.

Zum Transport zu dem Teilsystem und/oder zur Speicherung an oder in diesem Teilsystem kann ein computerlesbares Medium, beispielsweise ein Memorystick, eine Festplatte oder ein sonstiger transportabler oder fest eingebauter Datenträger dienen, auf welchem die von einer Rechnereinheit einlesbaren und ausführbaren Programmabschnitte des Computerprogramms gespeichert sind. Die Rechnereinheit kann z. B. hierzu einen oder mehrere zusammenarbeitende Mikroprozessoren oder dergleichen aufweisen. Die Rechnereinheit kann zum Beispiel Teil eines Terminals oder einer Steuereinrichtung eines bildgebenden Systems, wie zum Beispiel einer CT-Anlage, sein, sie kann aber auch Teil eines entfernt positionierten Serversystems innerhalb eines Datenübertragungsnetzes sein, das mit dem bildgebenden System kommuniziert.

Die abhängigen Ansprüche sowie die nachfolgende Beschreibung enthalten jeweils besonders vorteilhafte Ausgestaltungen und Weiterbildungen der Erfindung. Dabei können insbesondere die Ansprüche einer Anspruchskategorie auch analog zu den abhängigen Ansprüchen einer anderen Anspruchskategorie weitergebildet sein. Zudem können im Rahmen der Erfindung die verschiedenen Merkmale unterschiedlicher Ausführungsbeispiele und Ansprüche auch zu neuen Ausführungsbeispielen kombiniert werden.

Besonders vorteilhaft ist es, wenn das Spektrum der Röntgenabsorption des erfindungsgemäßen Röntgenkontrastmittels dem Spektrum der Röntgenabsorption von Wasser oder Weichgewebe ähnlich ist. Denn Wasser oder Weichgewebe weisen in einem für die Angiographie relevanten Energiebereich ein von der Röntgenphotonenenergie unabhängiges Verhalten auf und lassen sich daher leicht von anderen Körpermaterialien, wie zum Beispiel Calcium, trennen.

In einer besonders vorteilhaften Ausgestaltung der Erfindung weist das erfindungsgemäße Röntgenkontrastmittel eines der folgenden Materialien auf:
- Wolfram,
- Tantal,
- Hafnium,
- Gold.

Die genannten Materialien weisen alle vorteilhaft ein wasser-ähnliches Absorptionsverhalten auf und lassen sich daher leicht von Calcium-haltigen eines Untersuchungsbereichs trennen bzw. getrennt darstellen.

In einer Ausgestaltung des erfindungsgemäßen Röntgenbildgebungsverfahrens weist dieses ein Multi-Energie-Bildgebungsverfahren, vorzugsweise ein Dual-Energie-Bildgebungsverfahren, auf. Bei dem Multi-Energie-Bildgebungsverfahren werden mindestens zwei unterschiedliche Röntgenröhrenspannungen, bei denen sich die Änderung der Röntgenabsorption des erfindungsgemäßen Kontrastmittels von der Röntgenabsorption von Calcium signifikant unterscheidet, festgelegt. Weiterhin werden mindestens zwei Röntgenbildaufnahmen mit den mindestens zwei unterschiedlichen Röntgenröhrenspannungen zur Akquisition eines ersten Rohdatensatzes und mindestens eines zweiten Rohdatensatzes durchgeführt. Die Materialzerlegung erfolgt dann auf Basis der mindestens zwei Rohdatensätze. Bei dieser Variante werden mit Hilfe unterschiedlicher Röntgenröhrenspannungen Röntgenstrahlen mit unterschiedlichen Röntgenspektren erzeugt, die zur Erzeugung von mindestens zwei Rohdatensätzen, welche zur Trennung unterschiedlicher Materialien bei der Bildgebung verwendet werden, genutzt werden.

In einer alternativen Ausgestaltung des erfindungsgemäßen Röntgenbildgebungsverfahrens werden Röntgenrohdaten, die mit Hilfe eines photonenzählenden Detektors energieaufgelöst aufgenommen wurden, erfasst, wobei die Energieschwellen des photonenzählenden Detektors derart festgelegt werden, dass sich bei den durch die Energieschwellen festgelegten unterschiedlichen Energiebereichen die Änderung der Röntgenabsorption des erfindungsgemäßen Kontrastmittels von der Änderung der Röntgenabsorption von Calcium signifikant unterscheidet. Weiterhin erfolgt eine Materialzerlegung auf Basis der energieaufgelösten Rohdaten. Vorteilhaft wird bei dieser Variante nur die Bestrahlung eines Untersuchungsbereichs mit nur einer Röntgenröhre benötigt.

Die Erfindung wird im Folgenden unter Hinweis auf die beigefügten Figuren anhand von Ausführungsbeispielen noch einmal näher erläutert. Es zeigen:
FIG 1 ein Schaubild, welches die Absorptionseigenschaften des Kontrastmittels Iod und des Knochenmaterials Calcium in Abhängigkeit von der Energie der Röntgenphotonen veranschaulicht,
FIG 2 ein Schaubild, welches Absorptionswerte des Kontrastmittels Iod und des Materials Wolfram in Abhängigkeit von der Röhrenspannung eines Röntgengeräts veranschaulicht,
FIG 3 ein Schaubild, welches Absorptionseigenschaften der Kontrastmittel Iod und Wolfram sowie von Calcium und Wasser in Abhängigkeit von der Energie der Röntgenphotonen darstellt,
FIG 4 ein Flussdiagramm, welches ein Röntgenbildgebungsverfahren gemäß einem Ausführungsbeispiel der Erfindung veranschaulicht,
FIG 5 eine schematische Darstellung einer Bildrekonstruktionseinrichtung gemäß einem Ausführungsbeispiel der Erfindung,
FIG 6 eine schematische Darstellung eines CT-Systems gemäß einem Ausführungsbeispiel der Erfindung.

In FIG 1 ist ein Schaubild 10 gezeigt, welches die Absorptionseigenschaften des Kontrastmittels Iod I und des Knochenmaterials Calcium Ca in Abhängigkeit von der Energie E_{PH} der Röntgenphotonen veranschaulicht. Zur Veranschaulichung der Absorption der genannten Materialien ist der Massenabsorptionskoeffizient κ in Abhängigkeit von der Energie E_{PH} der Röntgenphotonen aufgetragen. Weiterhin ist in FIG 1 eine typische mittlere Energie E(1) einer Bildaufnahme mit niedriger Energie und die mittlere Energie E(2) einer Bildaufnahme mit hoher Energie einer Dual-Energie-Bildaufnahme gezeigt. Wie in FIG 1 zu erkennen ist, ist der Verlauf der beiden Kurven der Massenabsorptionskoeffizienten von Iod I und Calcium Ca recht ähnlich. Dabei muss berücksichtigt werden, dass die Materialien Iod und Calcium in unterschiedlicher Dichte und Konzentration vorliegen können. Dies führt dazu, dass die in FIG 1 gezeigten Absorptionskurven im ungünstigsten Fall vollständig übereinander liegen. Eine bildliche Trennung der beiden Materialien ist dann nicht mehr möglich.

In FIG 2 ist ein Schaubild 20 gezeigt, welches Absorptionswerte Iₛ des Kontrastmittels Iod I und des Materials Wolfram W in Abhängigkeit von der Röhrenspannung V_{T} eines Röntgengeräts veranschaulicht. Während die Röntgenabsorption von Iod mit zunehmender Energie abnimmt, ändert sich die Röntgenabsorption von Wolfram W mit der Energie nur wenig. Insbesondere bei einer Dual-Energie-Bildaufnahme bei einer niedrigen Energie von 80 kV und einer höheren Energie von 140 kV oder 150 kV mit Zinnfilter verändert sich die Röntgenabsorption Iₛ von Wolfram W im Vergleich zu der Röntgenabsorption von Iod praktisch nicht. Mithin lassen sich Bildpunkte, bei denen die beiden Einzelaufnahmen mit unterschiedlichen Röhrenspannungen erzeugt werden, leicht einem der beiden Kontrastmittel zuordnen. Beispielsweise ist ein Punkt, bei dem die Absorption in den beiden Bildern gleich ist, eindeutig dem Material Wolfram zuzuordnen und ein Punkt, bei dem die Absorption in den beiden Bildern stark unterschiedlich ist, eindeutig dem Material Iod zuzuordnen.

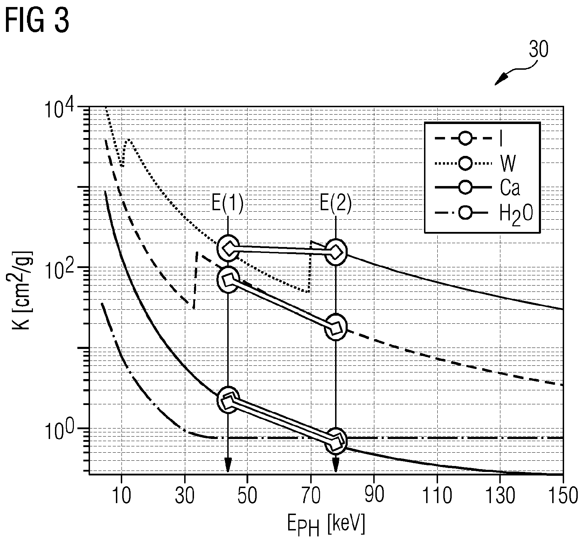

In FIG 3 ist ein Schaubild 30 dargestellt, welches Absorptionseigenschaften der Kontrastmittel Iod I und Wolfram W sowie von Calcium Ca und Wasser H₂O in Abhängigkeit von der Energie E_{PH} der Röntgenphotonen veranschaulicht. Für die genannten Materialien ist jeweils der Massenabsorptionskoeffizient κ in Abhängigkeit von der Energie E_{PH} der Röntgenphotonen aufgetragen. In FIG 3 ist gut zu erkennen, dass die Absorption des Kontrastmittels Iod I und des Knochenmaterials Calcium Ca in den Bereichen von 40 bis 80 keV mit zunehmender Photonenenergie E_{PH} stark abnimmt. Dabei ist zu beachten, dass die Absorption logarithmisch dargestellt ist. Im Gegensatz dazu verhält sich Wolfram W eher wie Wasser H₂O. D.h., die Absorption von Wolfram W ist für eine erste Photonenenergie E(1), die ungefähr bei 45 keV liegt, gleich der Absorption bei einer zweiten Photonenenergie E(2), welche ungefähr bei 80 keV liegt. Aufgrund des stark unterschiedlichen Verhaltens von Wolfram W gegenüber Calcium Ca, können Bildbereiche, welche mit Wolfram W beaufschlagt sind, leicht von Bereichen, in denen Calcium Ca vorherrscht, getrennt werden bzw. getrennt dargestellt werden.

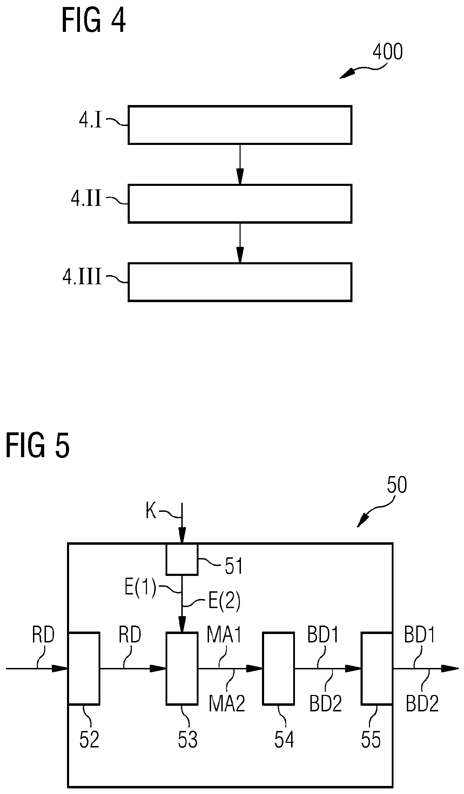

In FIG 4 ist ein Flussdiagramm 400 gezeigt, welches ein Röntgenbildgebungsverfahren gemäß einem Ausführungsbeispiel der Erfindung veranschaulicht. Bei dem Schritt 4.I wird zunächst ein auf dem Element Wolfram basierendes Kontrastmittel für eine angiographische Bildgebung von einem Untersuchungsbereich eines Patienten, beispielsweise dem Schädel des Patienten, ausgewählt. Weiterhin werden bei dem Schritt 4.II Röntgenrohdaten RD, die von einem von dem ausgewählten Kontrastmittel durchfluteten Bereich eines Untersuchungsobjekts O mit Hilfe eines Dual-Energie-Aufnahmeverfahrens aufgenommen wurden, erfasst. In dem in FIG 4 veranschaulichten Verfahren werden dabei Röntgenrohdaten mit Röntgenstrahlen, die mit zwei unterschiedlichen Energiewerten E(1) und E(2) aufgenommen wurden, erfasst. Die Energiewerte werden dabei so gewählt, dass das Absorptionsverhalten des ausgewählten Kontrastmittels, in diesem Ausführungsbeispiel ein auf dem Material Wolfram basierendes Kontrastmittel, für beide Energiewerte gleich ist. Dieser Vorgang kann zum Beispiel durch den Einsatz von zwei räumlich voneinander getrennt angeordneten Detektoren realisiert werden, wobei vor einem der beiden Detektoren in den Strahlengang ein Filter eingebracht ist, der einen Teil des Spektrums der Röntgenstahlen herausfiltert. Es werden also zwei Rohdatensätze erfasst, denen unterschiedliche Röntgenenergien E(1), E(2) zugeordnet sind.

Bei dem Schritt 4.III erfolgt eine Rekonstruktion von zwei Bilddatensätzen BD1, BD2 auf Basis der beiden bei dem Schritt 4.II erzeugten Rohdatensätze. Dabei wird ein erster Bilddatensatz BD1 erzeugt, welcher einen durch das Kontrastmittel Wolfram beaufschlagten ersten Bildbereich darstellt, und ein zweiter Bilddatensatz BD2 erzeugt, welcher einen zu dem ersten Bildbereich komplementären zweiten Bildbereich darstellt, in dem calciumbasierte Strukturen sichtbar gemacht werden. Die Erzeugung der beiden Bilddatensätze BD1, BD2 kann zum Beispiel mit Hilfe einer Materialzerlegung auf Basis der bei dem Schritt 4.II akquirierten Rohdaten erfolgen.

In FIG 5 wird eine Rekonstruktionseinrichtung 50 gezeigt. Die Rekonstruktionseinrichtung 50 weist eine Ermittlungseinheit 51 auf. Die Ermittlungseinheit 51 empfängt Informationen hinsichtlich des zu verwendenden Kontrastmittel K und ermittelt Werte E(1), E(2) von zwei unterschiedlichen Röntgenphotonenenergien, bei denen sich ein ausgewähltes Kontrastmittel K wie Wasser verhält, d.h. die Absorption ist für beide Energiewerte gleich. Die von dem Kontrastmittel K zu trennenden, mit Calcium durchsetzten Bildbereiche dagegen weisen in von Röntgengeräten nutzbaren Energiebereichen eine deutliche spektrale Abhängigkeit der Absorption auf und lassen sich daher bei den ermittelten Energiewerten E(1), E(2) leicht von dem ausgewählten Kontrastmittel K unterscheiden. Die Wahl der Energiewerte E(1), E(2) kann zum Beispiel auf der Basis von in einem Datenspeicher abgespeicherten energieabhängigen Absorptionswerten des gewählten Kontrastmittels K erfolgen.

Die Wahl der Energiewerte E(1), E(2) kann im Rahmen eines Multi-Energie-Aufnahmeverfahrens bei der Wahl der Energien bzw. mittleren Energiewerte der zur Bildgebung verwendeten Röntgenquellen berücksichtigt werden. Werden zählende Detektoren zur Erfassung der Röntgenstrahlung verwendet, so können Energieschwellen bzw. Intervalle so gewählt werden, dass die genannten Energiewerte umfasst sind.

Die Rekonstruktionseinrichtung 50 weist auch eine Rohdatenempfangseinheit 52 zum Empfangen von Röntgenrohdaten RD auf. Die Rohdaten RD wurden mit Hilfe eines Dual-Energy-CT-Verfahrens von einem von dem Kontrastmittel K zumindest teilweise durchfluteten Bereich eines Untersuchungsobjekts akquiriert.

Die Rohdaten RD werden an eine Zerlegungseinheit 53 weitergeleitet, welche eine Materialzerlegung der Rohdaten RD auf Basis der Röntgenrohdaten RD bezüglich des Kontrastmittels K und Calcium durchführt. Die den einzelnen Absorptionsspektren der unterschiedlichen Materialien zugeordneten materialspezifischen Anteile MA1, MA2 der Rohdaten werden an eine Rekonstruktionseinheit 54 übermittelt, welche mindestens zwei Bilddatensätze BD1, BD2 auf Basis der materialspezifischen Anteile MA1, MA2 rekonstruiert. Ein erster Bilddatensatz BD1 veranschaulicht einen mit dem Kontrastmittel beaufschlagten ersten Bildbereich und ein zweiter Bilddatensatz BD2 veranschaulicht einen zu dem ersten Bildbereich komplementären zweiten Bildbereich, in dem mit Calcium behaftete Strukturen oder mit Iod kontrastierte Strukturen vorherrschen. Die erzeugten Bilddatensätze BD1, BD2 werden über eine Ausgangsschnittstelle 55 beispielsweise an eine Anzeigeeinheit, eine Datenspeichereinheit oder einen Steuerrechner mit Bildanzeige ausgegeben.

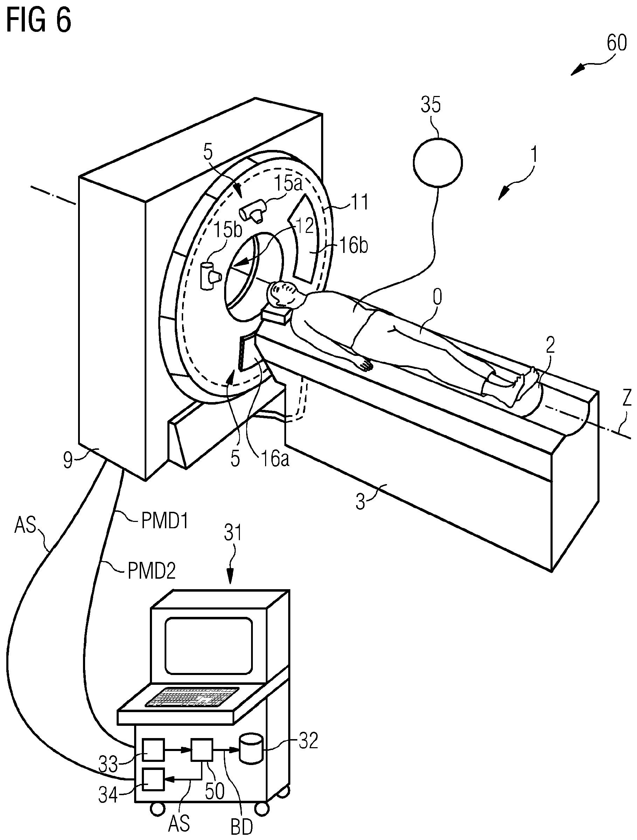

In FIG 6 ist ein Röntgenbildgebungssystem, in diesem Fall ein CT-System 60, gemäß einem Ausführungsbeispiel der Erfindung veranschaulicht.

Das CT-System 60, welches als Dual-Energie-CT-System ausgebildet ist, besteht dabei im Wesentlichen aus einem üblichen Scanner 9, in welchem an einer Gantry 11 eine Projektionsmessdatenakquisitionseinheit 5 mit zwei Detektoren 16a, 16b und zwei den Detektoren 16a, 16b gegenüberliegenden Röntgenquellen 15a, 15b um einen Messraum 12 umläuft. Vor dem Scanner 9 befindet sich eine Patientenlagerungseinrichtung 3 bzw. ein Patiententisch 3, dessen oberer Teil 2 mit einem darauf befindlichen Patienten O zum Scanner 9 verschoben werden kann, um den Patienten O durch den Messraum 12 hindurch relativ zum Detektorsystem 16a, 16b zu bewegen. Angesteuert werden der Scanner 9 und der Patiententisch 3 durch eine Steuereinrichtung 31, von der aus über eine übliche Steuerschnittstelle 34 Akquisitionssteuersignale AS kommen, um das gesamte System gemäß vorgegebener Messprotokolle in der herkömmlichen Weise anzusteuern. Im Fall einer Spiralakquisition ergibt sich durch eine Bewegung des Patienten O entlang der z-Richtung, welche der Systemachse z längs durch den Messraum 12 entspricht, und den gleichzeitigen Umlauf der Röntgenquellen 15a, 15b für die Röntgenquellen 15a, 15b relativ zum Patienten O während der Messung eine Helixbahn. Parallel laufen dabei immer gegenüber den Röntgenquellen 15a, 15b die Detektoren 16a, 16b mit, um Projektionsmessdaten PMD1, PMD2 zu erfassen, die dann zur Rekonstruktion von Volumen- und/oder Schicht-Bilddaten genutzt werden. Ebenso kann auch ein sequentielles Messverfahren durchgeführt werden, bei dem eine feste Position in z-Richtung angefahren wird und dann während eines Umlaufs, eines Teilumlaufs oder mehrerer Umläufe an der betreffenden z-Position die erforderlichen Projektionsmessdaten PMD1, PMD2 erfasst werden, um ein Schnittbild an dieser z-Position zu rekonstruieren oder um aus den Projektionsmessdaten mehrerer z-Positionen Bilddaten zu rekonstruieren. Das erfindungsgemäße Verfahren ist grundsätzlich auch an anderen CT-Systemen, z.B. mit nur einer Röntgenquelle oder einem einen vollständigen Ring bildenden Detektor, einsetzbar. Beispielsweise lässt sich das erfindungsgemäße Verfahren auch auf ein System mit unbewegtem Patiententisch und in z-Richtung bewegter Gantry (einer sogenannten Sliding Gantry) anwenden.

Die von den Detektoren 16a, 16b akquirierten Projektionsmessdaten PMD1, PMD2 (im Folgenden auch Rohdaten genannt) werden über eine Rohdatenschnittstelle 33 an die Steuereinrichtung 31 übergeben. Diese Rohdaten werden dann, gegebenenfalls nach einer geeigneten Vorverarbeitung in einer Rekonstruktionseinrichtung 50 weiterverarbeitet, die in diesem Ausführungsbeispiel in der Steuereinrichtung 31 in Form von Software auf einem Prozessor realisiert ist. Diese Rekonstruktionseinrichtung 50 rekonstruiert auf Basis der Rohdaten PMD1, PMD2 zwei Bilddatensätze BD1, BD2, von denen ein erster Bilddatensatz BD1 von einem erfindungsgemäßen Kontrastmittel K beaufschlagte Gefäßstrukturen veranschaulicht und ein zweiter Bilddatensatz BD2 Knochenstrukturen sowie kalzifizierte oder teilkalzifizierte Bereich in den Gefäßen darstellt.

Der genaue Aufbau einer solchen Rekonstruktionseinrichtung 50 ist in FIG 5 ausführlich dargestellt.

Die von der Rekonstruktionseinrichtung 50 erzeugten Bilddaten BD1, BD2 werden dann in einem Speicher 32 der Steuereinrichtung 31 hinterlegt und/oder in üblicher Weise auf dem Bildschirm der Steuereinrichtung 31 ausgegeben. Sie können auch über eine in FIG 6 nicht dargestellte Schnittstelle in ein an das Computertomographiesystem 60 angeschlossenes Netz, beispielsweise ein radiologisches Informationssystem (RIS), eingespeist und in einem dort zugänglichen Massenspeicher hinterlegt oder auf dort angeschlossenen Druckern oder Filming-Stationen als Bilder ausgegeben werden. Die Daten können so in beliebiger Weise weiterverarbeitet und dann gespeichert oder ausgegeben werden.

Zusätzlich ist in FIG 6 auch eine Kontrastmittel-Injektionseinrichtung 35 eingezeichnet, mit der dem Patienten O ein Kontrastmittel K vorab, d.h. vor dem Start des CT-Bildgebungsverfahrens injiziert wird. Die von dem Kontrastmittel K durchfluteten Bereiche sowie Knochenstrukturen und (teil-)kalzifizierte Bereiche können dann mit Hilfe des Computertomographiesystems 60 unter Anwendung des erfindungsgemäßen Röntgenbildgebungsverfahrens bildlich erfasst werden.

Die Komponenten der Rekonstruktionseinrichtung 50 können überwiegend oder vollständig in Form von Softwareelementen auf einem geeigneten Prozessor realisiert sein. Insbesondere können auch die Schnittstellen zwischen diesen Komponenten rein softwaremäßig ausgebildet sein. Erforderlich ist lediglich, dass Zugriffsmöglichkeiten auf geeignete Speicherbereiche bestehen, in denen die Daten geeignet zwischengelagert und jederzeit wieder aufgerufen und aktualisiert werden können.

Es wird abschließend noch einmal darauf hingewiesen, dass es sich bei den vorbeschriebenen Verfahren und Vorrichtungen lediglich um bevorzugte Ausführungsbeispiele der Erfindung handelt und dass die Erfindung vom Fachmann variiert werden kann, ohne den Bereich der Erfindung zu verlassen, soweit er durch die Ansprüche vorgegeben ist. Es wird der Vollständigkeit halber auch darauf hingewiesen, dass die Verwendung der unbestimmten Artikel "ein" bzw. "eine" nicht ausschließt, dass die betreffenden Merkmale auch mehrfach vorhanden sein können. Ebenso schließt der Begriff "Einheit" nicht aus, dass diese aus mehreren Komponenten besteht, die gegebenenfalls auch räumlich verteilt sein können.

## Patentansprüche

1. Röntgenkontrastmittel (K) zur Darstellung von mit dem Röntgenkontrastmittel (K) durchfluteten Blutgefäßen, aufweisend eine Röntgenabsorption, deren Änderung zwischen mindestens zwei unterschiedlichen Röntgenphotonenenergien (E(1), E(2)) sich signifikant von der Änderung der Röntgenabsorption von Calcium zwischen den mindestens zwei unterschiedlichen Röntgenphotonenenergien (E(1), E(2)) unterscheidet, wobei die Röntgenabsorption für die mindestens zwei Röntgenphotonenenergien (E(1), E(2)) nicht signifikant unterschiedlich ist.

2. Röntgenkontrastmittel nach Anspruch 1, wobei das Spektrum der Röntgenabsorption des Röntgenkontrastmittels (K) dem Spektrum der Röntgenabsorption von Wasser oder Weichgewebe ähnlich ist.

3. Röntgenkontrastmittel nach einem der vorstehenden Ansprüche, aufweisend eines der folgenden Materialien:
- Wolfram,
- Tantal,
- Hafnium,
- Gold.

4. Röntgenbildgebungsverfahren zur Darstellung von mit einem Röntgenkontrastmittel (K) durchfluteten Blutgefäßen, aufweisend die Schritte:
- Wahl eines Kontrastmittels (K) nach einem der vorstehenden Ansprüche,
- Erfassen von Röntgenrohdaten (RD, PMD1, PMD2) von einem von dem Kontrastmittel (K) durchfluteten Bereich eines Untersuchungsobjekts (O) mit Hilfe eines Multi-Energie-Aufnahmeverfahrens,
- Durchführen einer Materialzerlegung auf Basis der Röntgenrohdaten (RD, PMD1, PMD2) bezüglich des Kontrastmittels (K) und Calcium,
- Rekonstruieren von mindestens zwei Bilddatensätzen (BD1, BD2) auf Basis der Materialzerlegung, umfassend:
- einen ersten Bilddatensatz (BD1), welcher einen durch das Kontrastmittel (K) beaufschlagten ersten Bildbereich darstellt,
- einen zweiten Bilddatensatz (BD2), welcher einen vorzugswiese zu dem ersten Bildbereich komplementären zweiten Bildbereich darstellt.

5. Röntgenbildgebungsverfahren nach Anspruch 4, aufweisend ein Multi-Energie-Bildgebungsverfahren, mit den Schritten:
- Festlegen von mindestens zwei unterschiedlichen Röntgenröhrenspannungen (V_{T}), bei denen sich eine Änderung der Absorption des Kontrastmittels (K) von Calcium signifikant unterscheidet,
- Erfassen von mindestens zwei Datensätzen von Röntgenbildaufnahmen, die mit den mindestens zwei unterschiedlichen Röntgenröhrenspannungen (V_{T}) aufgenommen wurden, zur Akquisition eines ersten Rohdatensatzes (PMD1) und mindestens eines zweiten Rohdatensatzes (PMD2),
- Durchführen der Materialzerlegung auf Basis der mindestens zwei Rohdatensätze (PMD1, PMD2).

6. Röntgenbildgebungsverfahren nach Anspruch 4, aufweisend die Schritte:
- ernergieaufgelöstes Erfassen von Röntgenrohdaten (RD, PMD1, PMD2) mit Hilfe eines photonenzählenden Detektors, wobei die Energieschwellen des photonenzählenden Detektors derart festgelegt werden, dass sich bei diesen die Änderung der Röntgenabsorption des Röntgenkontrastmittels (K) von der Änderung der Röntgenabsorption von Calcium signifikant unterscheidet,
- Durchführen der Materialzerlegung auf Basis der energieaufgelösten Rohdaten (RD, PMD1, PMD2).

7. Röntgenbildgebungsverfahren nach einem der Ansprüche 4 bis 6, aufweisend ein CT-angiographisches Bildgebungsverfahren.

8. Bildrekonstruktionseinrichtung (50), aufweisend:
- eine Ermittlungseinheit (51) zum Ermitteln von mindestens zwei unterschiedlichen Röntgenphotonenenergien (E(1), E(2)), bei denen sich ein Röntgenkontrastmittel (K) nach einem der Ansprüche 1 bis 3 signifikant von der Änderung der Röntgenabsorption von Calcium zwischen den mindestens zwei unterschiedlichen Röntgenphotonenenergien (E(1), E(2)) unterscheidet,
- eine Rohdatenempfangseinheit (52) zum Empfangen von Röntgenrohdaten (RD) von einem von dem Röntgenkontrastmittel (K) zumindest teilweise durchfluteten Bereich eines Untersuchungsobjekts (O) mit Hilfe eines Multi-Energie-Aufnahmeverfahrens,
- eine Zerlegungseinheit (53) zum Durchführen einer Materialzerlegung auf Basis der Röntgenrohdaten (RD, PMD1, PMD2) bezüglich des Röntgenkontrastmittels (K) und Calcium,
- eine Rekonstruktionseinheit (54) zum Rekonstruieren von mindestens zwei Bilddatensätzen (BD1, BD2) auf Basis der Materialzerlegung, umfassend:
- einen ersten Bilddatensatz (BD1), welcher einen durch das Röntgenkontrastmittel (K) beaufschlagten ersten Bildbereich darstellt,
- einen zweiten Bilddatensatz (BD2), welcher einen vorzugsweise zu dem ersten Bildbereich komplementären zweiten Bildbereich darstellt.

9. Röntgenbildgebungssystem (60), aufweisend eine Bildrekonstruktionseinrichtung (50) nach Anspruch 8.

10. Röntgenbildgebungssystem nach Anspruch 9, aufweisend eine CT-Angiographieeinrichtung.

11. Computerprogrammprodukt mit einem Computerprogramm, welches direkt in eine Speichereinrichtung eines Röntgenbildgebungssystems (60) ladbar ist, mit Programmabschnitten, um alle Schritte eines Verfahrens nach einem der Ansprüche 4 bis 7 auszuführen, wenn das Computerprogramm in dem Röntgenbildgebungssystem (60) ausgeführt wird.

12. Computerlesbares Medium, auf welchem von einer Rechnereinheit einlesbare und ausführbare Programmabschnitte gespeichert sind, um alle Schritte eines Verfahrens nach einem der Ansprüche 4 bis 7 auszuführen, wenn die Programmabschnitte von der Rechnereinheit ausgeführt werden.

## Claims

1. X-ray contrast agent (K) for the representation of blood vessels flooded with the X-ray contrast agent (K), having an X-ray absorption the change of which between at least two different X-ray photon energies (E(1), E(2)) differs significantly from the change in the X-ray absorption of calcium between the at least two different X-ray photon energies (E(1), E(2)), wherein the X-ray absorption for the at least two X-ray photon energies (E(1), E(2)) is not significantly different.

2. X-ray contrast agent according to claim 1, wherein the spectrum of the X-ray absorption of the X-ray contrast agent (K) is similar to the spectrum of the X-ray absorption of water or soft tissue.

3. X-ray contrast agent according to one of the preceding claims, having one of the following materials:
- tungsten,
- tantalum,
- hafnium,
- gold.

4. X-ray imaging method for the representation of blood vessels flooded with an X-ray contrast agent (K), having the steps:
- selecting a contrast agent (K) according to one of the preceding claims,
- capturing X-ray raw data (RD, PMD1, PMD2) from a region of an examination object (0) which is flooded by the contrast agent (K), with the aid of a multi-energy recording method,
- carrying out a material decomposition on the basis of the X-ray raw data (RD, PMD1, PMD2) in relation to the contrast agent (K) and calcium,
- reconstructing at least two image datasets (BD1, BD2) on the basis of the material decomposition, comprising:
- a first image dataset (BD1) which represents a first image region affected by the contrast agent (K),
- a second image dataset (BD2) which represents a second image region which is preferably complementary to the first image region.

5. X-ray imaging method according to claim 4, having a multi-energy imaging method with the steps:
- specifying at least two different X-ray tube voltages (V_{T}) at which a change in the X-ray absorption of the contrast agent (K) significantly differs from calcium,
- capturing at least two datasets of X-ray image recordings that have been recorded with the at least two different X-ray tube voltages (V_{T}) for acquisition of a first raw dataset (PMD1) and at least one second raw data set (PMD2),
- carrying out the material decomposition on the basis of the at least two raw datasets (PMD1, PMD2).

6. X-ray imaging method according to claim 4, having the steps:
- capture of X-ray raw data (RD, PMD1, PMD2) with the aid of a photon-counting detector in an energy-resolved manner, wherein the energy thresholds of the photon-counting detector are set such that therewith, the change in the X-ray absorption of the X-ray contrast agent (K) differs significantly from the change in the X-ray absorption of calcium,
- carrying out the material decomposition on the basis of the energy-resolved raw data (RD, PMD1, PMD2).

7. X-ray imaging method according to one of claims 4 to 6, having a CT angiographic imaging method.

8. Image reconstruction facility (50), having:
- an ascertaining unit (51) for ascertaining at least two different X-ray photon energies (E(1), E(2)) at which an X-ray contrast agent (K) according to one of claims 1 to 3 differs significantly from the change in the X-ray absorption of calcium between the at least two different X-ray photon energies (E(1), E(2)),
- a raw data receiving unit (52) for receiving X-ray raw data (RD) from a region of an examination object (0) which is partially flooded by the X-ray contrast agent (K), with the aid of a multi-energy recording method,
- a decomposition unit (53) for carrying out a material decomposition on the basis of the X-ray raw data (RD, PMD1, PMD2) in relation to the X-ray contrast agent (K) and calcium,
- a reconstruction unit (54) for reconstructing at least two image datasets (BD1, BD2) on the basis of the material decomposition, comprising:
- a first image dataset (BD1) which represents a first image region affected by the X-ray contrast agent (K),
- a second image dataset (BD2) which represents a second image region which is preferably complementary to the first image region.

9. X-ray imaging system (60), having an image reconstruction facility (50) according to claim 8.

10. X-ray imaging system according to claim 9, having a CT angiography facility.

11. Computer program product having a computer program which can be directly loaded into a storage facility of an X-ray imaging system (60), having program portions in order to carry out all the steps of a method according to one of claims 4 to 7 when the computer program is executed in the X-ray imaging system (60).

12. Computer-readable medium on which program portions that can be read in and executed by a computer unit are stored, in order to carry out all the steps of a method according to one of claims 4 to 7 when the program portions are executed by the computer unit.

## Revendications

1. Agent (K) de contraste aux rayons X pour la représentation de vaisseaux sanguins traversés par l'agent (K) de contraste aux rayons X, ayant une absorption de rayons X, dont la variation entre au moins deux énergies (E(1), E(2)) photoniques de rayons X différentes se distingue significativement de la variation de l'absorption de rayons X du calcium entre les au moins deux énergies (E(1), E(2)) photoniques de rayons X différentes, dans lequel l'absorption de rayons X pour les au moins deux énergies (E(1), E(2)) photoniques de rayons X n'est pas différente significativement.

2. Agent de contraste aux rayons X suivant la revendication 1, dans lequel le spectre de l'absorption de rayons X de l'agent (K) de contraste aux rayons X est semblable au spectre de l'absorption de rayons X de l'eau ou du tissu mou.

3. Agent de contraste aux rayons X suivant l'une des revendications précédentes, comportant l'un des matériaux suivants :
- tungstène,
- tantale,
- hafnium,
- or.

4. Procédé d'imagerie par rayons X pour la représentation de vaisseaux sanguins traversés par un agent (K) de contraste aux rayons X, comportant les stades :
- choix d'un agent (K) de contraste suivant l'une des revendications précédentes,
- saisie de données (RD, PMD1, PMD2) brutes de rayons X d'une partie traversée par l'agent (K) de contraste d'un objet (0) à examiner, à l'aide d'un procédé d'enregistrement à plusieurs énergies,
- exécution d'une décomposition de matière sur la base des données (RD, PMD1, PMD2) brutes de rayons X en ce qui concerne l'agent (K) de contraste et le calcium,
- reconstruction d'au moins deux ensembles (BD1, BD2) de données d'image sur la base de la décomposition de matière, comprenant :
- un premier ensemble (BD1) de données d'image, qui représente une première zone d'image alimentée en agent (K) de contraste,
- un deuxième ensemble (BD2) de données d'image, qui représente une deuxième zone d'image, de préférence complémentaire de la première zone d'image.

5. Procédé d'imagerie par rayons X suivant la revendication 4, comportant un procédé d'imagerie à plusieurs énergies, comprenant les stades :
- fixation d'au moins deux tensions (V_{T}) de tubes de rayons X différentes, pour lesquelles une variation de l'absorption de l'agent (K) de contraste se distingue significativement de celle du calcium,
- saisie d'au moins deux ensembles de données d'enregistrement d'image aux rayons X, qui ont été enregistrés au au moins deux tensions (V_{T}) de tubes de rayons X différentes, pour l'acquisition d'un premier ensemble (PMD1) de données brutes et d'au moins un deuxième ensemble (PMD2) de données brutes,
- exécution de la décomposition de matière sur la base des au moins deux ensembles (PMD1, PMD2) de données brutes.

6. Procédé d'imagerie par rayons X suivant la revendication 4, comportant les stades :
- saisie avec résolution d'énergie de données (RD, PMD1, PMD2) brutes de rayons X, à l'aide d'un détecteur comptant les photons, dans lequel on fixe les seuils d'énergie du détecteur comptant les photons, de manière en ce que, pour ceux-ci, la variation de l'absorption de rayons X de l'agent (K) de contraste aux rayons X se distingue significativement de l'absorption de rayons X par le calcium,
- exécution de la décomposition de matière sur la base des données (RD, PMD1, PMD2) brutes avec résolution d'énergie.

7. Procédé d'imagerie par rayons X suivant l'une des revendications 4 à 6, comportant un procédé d'imagerie angiographique de tomodensitométrie.

8. Dispositif (50) de reconstruction d'image, comportant :
- une unité (51) de détermination pour la détermination d'au moins deux énergies (E(1), E(2)) photoniques de rayons X différentes, pour lesquelles un agent (K) de contraste aux rayons X suivant l'une des revendications 1 à 3 se distingue significativement de la variation de l'absorption de rayons X par le calcium entre les au moins deux énergies (E(1), E(2)) photoniques de rayons X différentes,
- une unité (52) de réception de données brutes pour la réception de données (RD) brutes de rayons X d'une zone traversée au moins en partie par l'agent (K) de contraste aux rayons X d'un objet (O) à étudier, à l'aide d'un procédé d'enregistrement à plusieurs énergies,
- une unité (53) de décomposition pour effectuer une décomposition de matière sur la base des données (RD, PMD1, PMD2) brutes de rayons X en ce qui concerne l'agent (K) de contraste aux rayons X et le calcium,
- une unité (54) de reconstruction pour la reconstruction d'au moins deux ensembles (BD1, BD2) de données d'image sur la base de la décomposition de matière comprenant :
- un premier ensemble (BD1) de données d'image, qui représente une première zone d'image alimentée en agent (K) de contraste,
- un deuxième ensemble (BD2) de données d'image, qui représente une deuxième zone d'image, de préférence complémentaire de la première zone d'image.

9. Système (60) d'imagerie par rayons X, comportant un dispositif (50) de reconstruction d'image suivant la revendication 8.

10. Système d'imagerie par rayons X suivant la revendication 9, comportant un dispositif angiographique par tomodensitométrie.

11. Produit de programme d'ordinateur comprenant un programme d'ordinateur, qui peut être chargé directement dans un dispositif de mémoire d'un système (60) d'imagerie par rayons X, comprenant des parties de programme pour exécuter tous les stades d'un procédé suivant l'une des revendications 4 à 7, lorsque le programme d'ordinateur est exécuté dans le système (60) d'imagerie par rayons X.

12. Support exécutable par ordinateur, sur lequel sont mises en mémoire des parties de programme pouvant être lues et exécutées par une unité informatique, afin d'exécuter tous les stades d'un procédé suivant l'une des revendications 4 à 7, lorsque les parties de programme sont exécutées par l'unité informatique.
